# EUROPEAN PATENT SPECIFICATION

(11) **EP 0 638 806 B1**
(45) Date of publication and mention of the grant of the patent: **26.04.2000**
(21) Application number: 94202290.6
(22) Date of filing: 09.08.1994
(51) Int. Cl.: G01N 33/53, G01N 33/569, G01N 33/543

(54) **Immunoassay method**
Immunoassay-Verfahren
Méthode d'immunoessai

(30) Priority: 12.08.1993 GB 9316743
(43) Date of publication of application: 15.02.1995
(73) Proprietor: KODAK LIMITED, Harrow, Middlesex HA1 4TY (GB); Johnson & Johnson Clinical Diagnostics, Inc., Rochester New York 14650 (US)
(72) Inventor: Glover, Justin Mark, c/o Kodak Limited, Harrow, Middlesex, HA1 4TY (GB); Jones, Mark William, c/o Kodak Limited, Harrow, Middlesex, HA1 4TY (GB)
(74) Representative: Mercer, Christopher Paul

(56) References cited:
- EP-A- 0 038 181
- EP-A- 0 080 109
- EP-A- 0 148 166
- US-A- 4 313 927
- US-A- 4 877 726

## Description

This invention relates to an immunoassay method for the determination of a specific substance or analyte in a liquid sample and to a kit which can be used to make the determination.

In many immunoassays saturation of the solid phase by antibody or antigen limits the effective measurement range of the assay by reducing the slope of the dose response profile at high analyte concentrations.

A further problem with immunoassays undertaken on microtitre plates is that they are often subject to excessive assay drift due to the time taken to pipette samples into anything up to 96 wells. Analyte in the first sample added is able to react with the antibody or antigen on the solid phase for a longer period than that in the last sample added. This can introduce a systematic bias into the results obtained giving an effect known as assay drift. Assay drift is a problem particularly in assays where rapid binding of antibody to antigen takes place. To date there has been no fully effective solution to this problem apart from the limitation of the number of samples assayed at the same time, i.e. by limiting to a half-plate or to a restricted number of strips of wells. In some instances it is simply accepted that assays are not completely effective because of assay drift.

Disassociating agents are known to prevent antigens binding to antibodies and this has been used in affinity chromatography. In a well-established affinity chromatography technique disassociating agents are used to separate antibodies or antigens from chromatography columns.

A number of proposals have been made to use chaotropic salts to improve various immunoassay techniques for example in US-A-5155023 and in DD-A-297010. US-A-5155023 discloses immunoreagents for the release, non-specific capture and detection of lipopolysaccharides that may be diagnostic for microorganisms causative for, among other things Chlamydia trachomatis or Chlamydia psittaci. DD-A-297010 discloses rosette inhibition test enhancer for early pregnancy factor by replacing guinea pig complement with a chaotropic anion, thereby reducing cost with standardisation.

European patent application EP-A-0 080 109 describes an immunoassay for antigens and antibodies wherein the antigen or antibody exists in the form of an immune complex. The assay described comprises dissociating the immunocomplex with a dissociating buffer, capturing the antigen or antibody on a solid support, washing the solid support, filling unoccupied sites on the solid support and carrying out an ELISA-type assay using an enzyme-labelled or radioactively-labelled antigen or antibody. In an alternative embodiment, the immunocomplex is captured on the solid support and then dissociated by washing with a suitable dissociating reagent.

European patent application EP-A-0 148 166 describes a method for detecting an antigen in a plasma or serum sample. The method comprises conditioning the sample (either by dilution or adding a dissociating reagent), drying and detecting the antigen in the dried sample using an immunological assay.

European patent application EP-A-0 038 181 describes an agglutination assay wherein the amount of antigen or antibody in a sample is determined by the extent to which they complex with and precipitate proteins bound to finely divided particles. In order to overcome-the interferences which arise from non-specific protein-protein interactions, a chaotropic (dissociating) reagent is added to the assay mixture, in a carefully controlled amount.

According to the present invention we provide an immunoassay method for the determination of an analyte in a liquid sample comprising a step in which the sample is contacted with assay reagents in a suitable container characterised in that the assay reagents include a disassociating agent added before or at the same time as the contact with the sample, the disassociating agent being one which (a) is capable of disassociating the antigen/antibody bond of the test to a significant extent, (b) is stable for the intended shelf life of the test reagents, (c) is soluble in the test reaction mixture and (d) does not interfere significantly with the reactions of the test when subsequently diluted with a second reagent.

The disassociating agent employed and the amount of it which is present during the assay will vary depending upon the characteristics of the assay system under consideration. Suitable disassociating agents are capable of disassociating the antibody/antigen bond of the test to the extent required when present in suitable amounts and are stable over considerable periods of time and certainly over at least the periods during which test reagents can reasonably be expected to be stored. Moreover suitable disassociating agents are soluble in the test reaction mixture and in solutions of the test reagents to at least an extent which is sufficient to enable effective amounts of the agents to be present in the reaction mixture and they are effectively inert to other assay components under the conditions of the reaction mixture and consequently do not interfere significantly with the subsequent reactions of the test under consideration.

Preferred disassociating agents include guanidine hydrochloride, chaotropic salts such as sodium dodecyl sulphate, potassium iodide, potassium cyanide and potassium thiocyanide and particularly urea. Depending upon the concentration of the disassociating agent in the reaction mixture of the assay under consideration, it can have the effect of slowing down, preventing or even reversing antigen/antibody binding. A suitable concentration will depend upon the characteristics of the assay system under consideration and can readily be determined by those technically qualified in this field. The disassociating agent can be introduced to the test in any suitable manner, for instance by inclusion in a diluent, in an assay reagent, as a separate reagent itself or by coating onto the walls of wells in which the assay is performed. The disassociating agent is added to the test reaction mixture at an appropriate time either before or at the same time as the sample to be assayed is introduced.

The problem of assay drift occurs particularly in assays where antibody/antigen binding occurs rapidly. It occurs in both quantitative and qualitative assays but has a more important effect in quantitative assays than in qualitative assays. Assays in which the method of the invention can be employed include, for example, rubella IgG assays, especially quantitative rubella IgG assays, for example the KODAK^{(TM)} AMERLITE^{(TM)} rubella IgG assay produced by Kodak Clinical Diagnostics Ltd., Amersham, UK and toxoplasma IgG assays, especially quantitative toxoplasma IgG assays, for example the KODAK AMERLITE Toxoplasma IgG assay also produced by Kodak Clinical Diagnostics Ltd. However the method can be usefully employed in a wide range of immunoassays.

The method of the invention will be further described as it is applied to a quantitative rubella IgG assay. It can however be applied to other assays and to qualitative assays. In the KODAK AMERLITE rubella IgG assay the materials used are as follows:-
1. A suitable number of coated wells (inactivated rubella antigen, binds >5 IU 2nd International Standard specific antibody per well). These are generally supplied in packs of 144 or 240.
2. 6 vials (0.5 ml each) of anti-rubella IgG Standards A-F (Standard A: bovine serum, Standards B-F: human plasma diluted in bovine serum) with Antimicrobial Agent. Nominal values of 0, 10, 50, 100, 200 and 500 IU/ml (2nd I.S.).
3. 1 bottle Sample Diluent (30 ml, yellow) in buffer with Antimicrobial Agent.
4. 2 bottles Assay Reagent (30 ml each, blue) in buffer with Antimicrobial Agent.
5. 1 vial Conjugate Concentrate (horseradish peroxidase-labelled sheep anti-human IgG, concentration >2,5 µg/ml, 0.6 ml, red) in buffer with Antimicrobial Agent.
6. 2 bottles Conjugate Diluent (17.1 ml each, red) in buffer with Antimicrobial Agent.

Using the materials listed above the KODAK AMERLITE quantitative rubella IgG assay is carried out as follows:-
1. A Shaker Incubator, Workstation, Washer and Analyzer are prepared for use.
2. A strip holder with sufficient wells to enable the running of all standards and specimens as required is assembled. If the last row does not contain a full complement of 12 coated wells it is completed with the clear blank wells provided separately.
3. The Standards are assayed in duplicate.
4. Standards and specimens are diluted in sample diluent (yellow). Suitable dilution protocol: 10 µl standard or specimen + 100 µl sample diluent. Diluted standards and specimens should be used immediately.
5. 10 µl diluted standard or specimen are pipetted into the appropriate wells. This is the step which gives rise to delay and can cause assay drift.
6. 200 µl of assay reagent (blue) are dispensed into each coated well.
7. The wells are covered with a clean lid then shaken and incubated at 37°C for 60 ± 5 minutes in the Shaker Incubator.
8. All wells are aspirated and washed using serology Wash Reagent in the Washer. Preferably the serology wash reagent is the KODAK AMERLITE serology wash reagent.
9. 100 µl of conjugate reagent (red) are dispensed into each coated well.
10. Wells are covered with lid then shaken and incubated at 37°C for 30 ± 2 minutes in the Shaker Incubator.
11. All wells are aspirated and washed using Serology Wash Reagent in the Washer.
12. 250 µl signal reagent are immediately dispensed into all the coated wells. Preferably the signal reagent is the AMERLITE signal reagent.
13. The wells are read in the Analyzer. Suitably this is done at a time within the range 2 to 20 minutes after addition of signal reagent.
14. The assay results are interpreted using the appropriate analyzer operator manual.

Preferably, as in step 5 above, samples are pre-diluted in a reagent (in this case the sample diluent) containing an appropriate concentration of disassociating agent, thus preventing any antigen-antibody binding in the antibody coated wells until the disassociating agent concentration is reduced by the rapid addition of the second diluent assay reagent. This reduces assay drift.

When the method of the invention is to be employed, the sample diluent (item 3 of the materials for the KODAK AMERLITE rubella IgG assay above) is modified to contain a disassociating agent, preferably urea, which is suitably present at a concentration in the range 20% to 50% and particularly 30% by weight of the sample diluent. This modified sample diluent may then be employed in step 4 of the KODAK AMERLITE quantitative rubella assay described above to give the assay method of the invention.

The method of the invention generally enables the effective measurement range of an assay to be increased and reduces assay drift, often considerably.

The invention is illustrated by the following Example:-

### Example

The KODAK AMERLITE rubella IgG quantitative assay was carried out according to steps 1 to 14 described above firstly using in step 4 a sample diluent of conventional type containing no disassociating agent and secondly using a sample diluent as previously described containing urea as a disassociating agent at a concentration of 30% by weight of the diluent. In both instances the assay was carried out by using 96 wells, an AMERLITE shaker incubator, AMERLITE workstation, AMERLITE washer and AMERLITE analyzer.

When using sample diluents without urea the diluted sample added to the first well reacted with the solid phase for approximately 20 minutes longer than the last sample added (time taken to pipette 96 samples), resulting in a significant degree of assay drift. Use of sample diluent containing urea prevented the antibody in the diluent sample reacting with the antigen on the solid phase. The reaction was triggered by the addition of an assay reagent which diluted the urea to a level at which it was no longer functional. Assay reagent can be added to a plate in a short time (approx. 2 minutes). Using the method of the invention assay drift was reduced approximately 3 fold. This is shown by the results in the Table below which gives measurements of rubella antibodies in International units per ml for the first and 96th samples in each experiment i.e. for samples tested at 0 and 20 minutes respectively.

**Table**

| Sample | Rubella antibody measurements in IU/ml:diluent without urea | | | Rubella antibody measurements in IU/ml:diluent with urea | | |
|---|---|---|---|---|---|---|
| | Sample 1 (0 mins) | Sample 96 (20 mins) | % drift | Sample 1 (0 mins) | Sample 96 (20 mins) | % drift |
| QCB | 18.13 | 8.97 | -50.5 | 9.7 | 8.1 | -16.7 |
| QCC | 58.5 | 27.9 | -52.4 | 28.2 | 23.6 | -16.2 |
| QCD | 440.3 | 301.0 | -31.6 | 334.3 | 312.3 | -6.6 |
| 110 | 316.3 | 188.0 | -40.6 | 179.0 | 151.3 | -15.5 |
| 111 | 294.0 | 190.3 | -35.3 | 210.3 | 197.0 | -6.3 |
| 113 | 127.3 | 73.3 | -42.4 | 62.97 | 65.37 | +3.8 |

The results in the Table are summarised below:-
Average drift (6 samples) : sample diluent without urea = -42.1%
Average drift (6 samples) : sample diluent with urea = -9.6%
Reduction in drift using urea according to the invention = >70%

The results in the Table show that use of a sample diluent containing urea significantly reduces assay drift.

## Claims

1. An immunoassay method for the determination of an analyte in a liquid sample comprising a first step in which the sample is contacted with at least one first assay reagent in a suitable container coated with a specific binding reagent and a second step in which at least one second assay reagent is added to the container to initiate immunocomplex formation, characterised in that the first assay reagent includes a disassociating agent added before or at the same time as the contact with the sample, the disassociating agent being one which (a) is capable of disassociating the antigen/antibody bond of the test to a significant extent in the first step, (b) is stable for the intended shelf life of the test reagents, (c) is soluble in the test reaction mixture and (d) does not interfere significantly with the reactions of the test when subsequently diluted with a second reagent in the second step.

2. The immunoassay of claim 1 in which the chaotropic agent is selected from the list comprising guanidine hydrochloride, sodium dodecyl sulphate, potassium iodide, potassium cyanide, potassium thiocyanate and urea.

3. An immunoassay method according to claims 1 or 2 characterised in that the assay is a quantitative assay.

4. An immunoassay method according to any one of claims 1 to 3 characterised in that the assay is a rubella IgG assay.

5. An immunoassay method according to any one of claims 1 to 3 characterised in that the assay is a toxoplasma IgG assay.

6. An immunoassay method according to any one of the preceding claims characterised in that the disassociating agent is urea.

7. An immunoassay method according to any one of claims 1 to 5 characterised in that the disassociating agent is guanidine hydrochloride or a chaotropic salt.

8. An immunoassay method according to any one of the preceding claims characterised in that the disassociating agent is added in a sample diluent.

9. An immunoassay method according to claim 8 characterised in that the disassociating agent is present in the sample diluent in an amount in the range 20% to 50% by weight of the sample diluent.

10. An immunoassay method according to claim 4 characterised in that the disassociating agent is present in the sample diluent in an amount of substantially 30% by weight of the sample diluent.

## Patentansprüche

1. Ein Immunoassay-Verfahren zur Bestimmung eines Analyten in einer flüssigen Probe, das einen ersten Schritt, in dem die Probe mit Wenigstens einem ersten Assay-Reagens in Kontakt gebracht wird in einem geeigneten Behälter, der mit einem spezifischen Bindungsreagens beschichtet ist, und einen zweiten Schritt umfaßt, in dem wenigstens ein zweites Assay-Reagens dem Behälter zugegeben wird, um Immunkomplex-Bildung zu initiieren, dadurch gekernzeichnet, daß das erste Assay-Reagens ein Disassoziationsagens einschließt, das vor oder zur selben Zeit wie der Kontakt mit der Probe zugegeben wird, wobei das Disassoziationsagens eines ist, das (a) in der Lage ist, die Antigen/Antikörper-Bindung des Tests zu einem signifikanten Ausmaß im ersten Schritt zu disassoziieren, (b) für die beabsichtigte Lagerungsdauer der Test-Reagentien stabil ist, (c) in der Testreaktions-Mischung löslich ist und (d) die Reaktionen des Tests nicht signifikant stört, wenn danach mit einem zweiten Reagens im zweiten Schritt verdünnt wird.

2. Immunoassay nach Anspruch 1, in dem das chaotrope Agens ausgewählt ist aus der Liste, die Guanidinhydrochlorid, Natriumdodecylsulfat, Kaliumiodid, Kaliumcyanid, Kaliumthiocyanat und Harnstoff umfaßt.

3. Immunoassay-Verfahren nach den Ansprüchen 1 oder 2, dadurch gekennzeichnet, daß der Assay ein quantitativer Assay ist.

4. Immunoassay-Verfahren nach einem der Ansprüche 1 bis 3, dadurch gekennzeichnet, daß der Assay ein Rubella-IgG-Assay ist.

5. Immunoassay-Verfahren nach einem der Ansprüche 1 bis 3, dadurch gekennzeichnet, daß der Assay ein Toxoplasma-IgG-Assay ist.

6. Immunoassay-Verfahren nach einem der vorangehenden Ansprüche, dadurch gekennzeichnet, daß das Disassoziationsagens Harnstoff ist.

7. Immunoassay-Verfahren nach einem der Ansprüche 1 bis 5, dadurch gekennzeichnet, daß das Disassoziationsagens Guanidinhydrochlorid oder ein chaotropes Salz ist.

8. Immunoassay-Verfahren nach einem der vorangehenden Ansprüche, dadurch gekennzeichnet, daß das Disassoziationsagens in einem Probenverdünnungsmittel zugegeben wird.

9. Immnnoassay-Verfahren nach Anspruch 8, dadurch gekennzeichnet, daß das Disassoziationsagens in dem Probenverdünnungsmittel in einer Menge im Bereich 20 % bis 50 %, in Gewicht des Probenverdunnungsmittels, vorliegt.

10. Immunoassay-Verfahren nach Anspruch 4, dadurch gekennzeichnet, daß das Disassoziationsagens in dem Probenverdünnungsmittel in einer Menge von im wesentlichen 30 %, in Gewicht des Probenverdünnungsmittels, vorliegt.

## Revendications

1. Procédé de dosage immunologique pour doser un analyte dans un échantillon liquide, comprenant une première étape dans laquelle l'échantillon est mis en contact avec au moins un premier réactif de dosage dans un récipient approprié revêtu d'un réactif de liaison spécifique et une seconde étape dans laquelle au moins un second réactif de dosage est ajouté au récipient pour amorcer la formation d'un complexe immunologique, caractérisé en ce que le premier réactif de dosage contient un agent de dissociation ajouté avant ou en même temps que la mise en contact avec l'échantillon, l'agent de dissociation étant un agent (a) capable de dissocier la liaison antigène/anticorps de l'essai à un degré significatif dans la première étape, (b) stable pendant la durée de conservation envisagée des réactifs d'essai, (c) soluble dans le mélange réactionnel d'essai et (d) sans interférence significative avec les réactions de l'essai lorsqu'il est dilué ensuite avec un second réactif dans la seconde étape.

2. Procédé de dosage immunologique selon la revendication 1, dans lequel l'agent chaotropique est choisi dans la liste comprenant le chlorhydrate de guanidine, le dodécylsulfate de sodium, l'iodure de potassium, le cyanure de potassium, le thiocyanure de potassium et l'urée.

3. Procédé de dosage immunologique selon la revendication 1 ou 2, caractérisé en ce que le dosage est un dosage quantitatif.

4. Procédé de dosage immunologique selon l'une quelconque des revendications 1 à 3, caractérisé en ce que le dosage est un dosage des IgG de la rubéole.

5. Procédé de dosage immunologique selon l'une quelconque des revendications 1 à 3, caractérisé en ce que le dosage est un dosage des IgG de la toxoplasmose.

6. Procédé de dosage immunologique selon l'une quelconque des revendications précédentes, caractérisé en ce que l'agent de dissociation est l'urée.

7. Procédé de dosage immunologique selon l'une quelconque des revendications 1 à 5, caractérisé en ce que l'agent de dissociation est le chlorhydrate de guanidine ou un sel chaotropique.

8. Procédé de dosage immunologique selon l'une quelconque des revendications précédentes, caractérisé en ce que l'agent de dissociation est ajouté dans un diluant pour échantillon.

9. Procédé de dosage immunologique selon la revendication 8, caractérisé en ce que l'agent de dissociation est présent dans le diluant pour échantillon à raison de 20% à 50% en poids du diluant pour échantillon.

10. Procédé de dosage immunologique selon la revendication 4, caractérisé en ce que l'agent de dissociation est présent dans le diluant pour échantillon essentiellement à raison de 30% en poids du diluant pour échantillon.
